# EUROPEAN PATENT SPECIFICATION

(11) **EP 3 232 958 B1**
(45) Date of publication and mention of the grant of the patent: **20.02.2019**
(21) Application number: 15804474.3
(22) Date of filing: 03.12.2015
(51) Int. Cl.: A61B 17/54, A61B 17/00, A61B 17/32, A61B 17/30

(54) **MICRODERMABRASION DEVICE**
VORRICHTUNG ZUR MIKROABTRAGUNG VON HAUTGEWEBE
DISPOSITIF DE MICRODERMABRASION

(30) Priority: 18.12.2014 EP 14198864
(43) Date of publication of application: 25.10.2017
(73) Proprietor: Koninklijke Philips N.V., 5656 AE Eindhoven (NL)
(72) Inventor: JURNA, Martin, 5656 AE Eindhoven (NL); BEIJENS, Linda Goverdina Maria, 5656 AE Eindhoven (NL); PEETERS, Felix Godfried Peter, 5656 AE Eindhoven (NL)
(74) Representative: Kabuk, Yavuz
(86) International application number: PCT/EP2015/078436
(87) International publication number: WO 2016/096444

(56) References cited:
- WO-A1-2011/006009
- US-A1- 2002 016 601
- US-A1- 2006 200 172
- US-A1- 2010 049 210
- US-A1- 2013 110 032

## Description

### FIELD OF THE INVENTION

The present invention relates to a device for microdermabrasive skin treatment.

### BACKGROUND OF THE INVENTION

Microdermabrasion is a well-accepted technology for skin treatment to improve the skin by addressing the first signs of aging. It is known that a microdermabrasion treatment results in several skin benefits including smooth skin, better overall appearance and even skin tone. Abrasion of the skin leads to smoothing of skin texture and improved radiance. By removing the top layers of the stratum corneum during a microdermabrasion treatment, signaling to the basal skin layer leads to an increased proliferation of viable keratinocytes and leads to a thickening and better alignment of the viable epidermal layer. Abrasion also has an effect on the skin's barrier function. This barrier function of the stratum corneum protects the body from penetration of bacteria or other substances and excessive water vaporization. A microdermabrasion treatment temporarily reduces the barrier function such that topicals may penetrate deeper into the skin and are more effective.

One way to improve the effectiveness of the treatment is to remove more layers of the stratum corneum by increasing the under-pressure, and thus increasing the abrasive contact area with the skin. However, such an approach may lead to unwanted skin reactions such as redness and dry skin.

US 2008/0249537 A1 describes a skin resurfacing device comprising a skin treater, a controllable vacuum source, and a skin sensor.

US2013/110032 A1 describes a system and method for treating a surface. According to the document, a flexible or adaptable pad for treating a surface may be particularly suitable for treatment of uneven surfaces. An apparatus may include a mounting section and a flexible surface comprising a plurality of treatment sections. The treatment sections may comprise an abrasive material. Cracks or grooves on a surface of the apparatus enable a first and second treatment sections to assume a respective first and second positions with respect to a treated surface. An edge of a groove may be designed to increase an abrasiveness of the apparatus and may remove material from a treated surface. Other embodiments are described and claimed.

A microdermabrasion device generally comprises a vacuum pump attached to a treatment head with an abrasive surface. The treatment head is moved over the surface of the skin resulting in a vacuum massage together with an exfoliation of dead skin cells, scraped off by the abrasive surface. Currently, such microdermabrasion devices generally only remove a few layers of the stratum corneum and keep the barrier function mostly intact. Consequently, after such treatment, topicals are still unable to penetrate to the deeper layers of the skin, and thus the effectiveness of the topicals is not enhanced by the treatment.

With the skin sensor of US 2008/0249537 A1 the skin type is determined and the vacuum generated by vacuum source is adapted in accordance with the detected skin type, such that an improved adjustment of the abrasion level is achieved. Adjustment is only controlled by the vacuum level, a fine adjustment of the abrasion level is not feasible.

Hence, there is a need for a new device which provides more control over the microdermabrasive treatment, in particular the abrasion level, and further enhances the treatment effectiveness.

### SUMMARY OF THE INVENTION

It is an object of the present invention to provide a microdermabrasion device which allows a fine-grained control over the treatment process to increase effectiveness of the treatment, in particular, enhanced topical penetration wherever necessary.

According to a first aspect of the present invention this object is achieved by a device for microdermabrasive treatment, comprising a treatment head having a cavity with inner walls, an opening in said inner walls, and a treatment head tip for placing the treatment head on a skin of the user, wherein the treatment head tip comprises a first abrasive surface and the inner walls comprise a second abrasive surface, wherein the opening is configured to be connected to a vacuum source for applying an under-pressure within the cavity when the treatment head tip is placed on the skin of the user, and wherein the first abrasive surface comprises a different roughness than the second abrasive surface, wherein the device is configured to have the second abrasive surface only contact the skin of the user when the under-pressure is applied to the cavity; and wherein the cavity has an essentially dome-like shape with an annular surrounding, and wherein the treatment head tip is formed by the annular surrounding, and the inner walls are formed by an inner surface of said dome-like shape, as further defined in the accompanying claims.

Preferred embodiments of the invention are defined in the dependent claims.

The present invention is based on the idea to enhance a microdermabrasive treatment by providing a treatment head with multiple abrasive surfaces each having a different roughness to allow within a single treatment the application of multiple abrasion levels. For that, the inner wall of the cavity is at least partly covered with abrasive particles, forming an abrasive surface having a different roughness than a further abrasive surface arranged at the treatment head tip. When the treatment head tip, which is preferably surrounding the cavity, is placed on the skin of the user, a volume is formed between the inner wall of the cavity and the skin of the user.

An under-pressure applied to said volume forces parts of the skin of the user to be drawn into the cavity and to be exposed to the abrasive surface which is arranged on the inner walls of the cavity. By moving the device over the skin of the user, the skin is at first exposed to the roughness of the abrasive surface of the treatment head tip, subsequently to the roughness of the inner wall, and finally again to the roughness of the treatment head tip.

Such a treatment leads to an intensified abrasive action, because the skin is exposed to different microdermabrasive intensities and may be further intensified by using variations in vacuum level to allow a fine-grained control over the abrasion levels.

Advantageously, multiple abrasion levels ranging from low intensity removal of the top skin layer up to high intensity treatment including a perforation of the stratum corneum of the skin of the user can be combined within a single device. Moreover, the multiple abrasion levels can be applied to different areas of the skin within a single treatment without interrupting the treatment itself.

Consequently, the new device can be used more flexibly for various skin types and treatment methods, and the treatment duration may be substantially shortened. Furthermore, a better control over the abrasion levels and the treatment itself may reduce the risk of undesired skin reaction such as redness and dehydration.

According to an embodiment of the present invention, a roughness of the second abrasive surface is higher than a roughness of the first abrasive surface.

In this case the roughness of the abrasive surface located within the cavity is higher than the roughness of the abrasive surface located at the treatment head tip. When an under-pressure is applied to the cavity after the treatment head tip has been placed onto the skin of a user, and the device is subsequently moved over the skin of the user, the skin is exposed to varying abrasion levels in a particular order. At first the skin is being exposed to the low roughness of the treatment head tip, subsequently the skin is exposed to the increased roughness of the abrasive surface of the inner wall, and finally the skin is exposed one more time to the (lower) roughness of the abrasive surface of the treatment head tip. The abrasive particles on the second abrasive surface have a higher roughness and may thus penetrate the skin further than the abrasive particles of the first abrasive surface. The skin will first feel a mild abrasion, followed by a more intense abrasion, followed once more by a mild abrasion. The application of the higher roughness of second abrasive surface enables a user to apply an intensified microdermabrasion with a reduced possibility of skin irritation.

According to a further embodiment of the present invention, the treatment head tip is arranged on a first side of the cavity and the opening is arranged on an opposite side of the cavity, wherein the roughness of the second abrasive surface increases towards the opening.

In this case the abrasiveness of the second abrasive surface varies and increases in roughness towards the opening. When the device is moved over the skin of a user and an under-pressure is applied to the cavity, the skin will be drawn towards the opening through which the under-pressure is applied. The abrasive action on a part of the skin thereby starts at a certain level, when the skin is in contact with the treatment head tip, and intensifies, when the part of the skin contacts the inner wall of the cavity. The abrasion effect further intensifies until the part of the skin passes the opening, where the roughness of the inner wall is highest. The part of the skin is subsequently exposed to a decreasing roughness and a smoothening experience, ending at the certain level of abrasiveness at the treatment head tip. This relieves the part of the skin and reduces the possibility of skin irritation.

According to yet another embodiment of the present invention, a roughness of the second abrasive surface is lower than a roughness of the first abrasive surface.

According to a further embodiment of the present invention, the first abrasive surface is arranged transverse to the second abrasive surface.

The second abrasive surface is thus arranged in an angle towards the first abrasive surface. The term "transverse" shall herein be understood as non-parallel and not necessarily as "perpendicular". The first abrasive surface is preferably arranged on a planar plane of the treatment head tip such that the first abrasive surface is in permanent contact with the user's skin when the treatment head tip is placed onto the user's skin. The second abrasive surface is arranged at an angle (non-parallel) to the first abrasive surface such that the user's skin contacts the second abrasive surface only when the skin is displaced by an under-pressure in the cavity and formed in to a skin dome having slopes corresponding to the angle between the first and second abrasive surface. This way, the appliance of the second abrasive surface can be easily controlled by the amount of under-pressure applied to the cavity. Hence, the first abrasive surface and second abrasive surface may have a mutual angle (especially in a plane also including an opening axis (see also below)) which is selected from the range of larger than 0° and smaller than 180°, especially selected from the range of 45-175°, such as especially selected from the range of 75-135°, such as about 90°. A transition from the first abrasive surface to the second abrasive surface may be bended and may include an edge. In embodiments, the first abrasive surface may substantially be planer and the second abrasive surface may substantially be planar or may be bended. When the second abrasive surface is bended, at least parts of the second abrasive surfac, such as at least 25%, like at least 50% of the area comprising the second abrasive surface may configured with said mutual angle relative to the first abrasive surface

According to a further embodiment of the present invention, the opening defines an opening axis, wherein the first abrasive surface and the second abrasive surface are arranged concentrically around said opening axis.

The opening defines thus the center of the abrasive surfaces and the opening axis defines the direction into which the skin of the user is drawn once an under-pressure is applied within the cavity. Therefore, the concentric arrangement causes the skin to be exposed to the first and second abrasive surface in substantially equal amounts when the device is moved at a steady speed over the user's skin. This way, a uniform abrasion by the first and second abrasive surface may be achieved.

According to a further embodiment of the present invention, the second abrasive surface is arranged closer to said opening axis than the first abrasive surface.

The second abrasive surface is thus arranged closer to the opening, and hence further within the cavity. Since the amount of skin that is drawn into the cavity depends on the amount of under-pressure applied to the cavity and skin properties such as elasticity, skin generally gets in contact with the abrasive surface further away from the opening first, and only afterwards with the abrasive surface arranged closer to the opening. By this arrangement the exposure of the user's skin to the abrasive surfaces can easily be controlled by simply adjusting the under-pressure within the cavity. Abrasion by the second abrasive surface is thus optionally, but occurs always after the skin has been treated by the first abrasive surface.

According to a further embodiment of the present invention, the treatment head tip and the cavity are arranged to form a single integral component.

As integral component the treatment head tip and the cavity may be produced as one piece and may be simply plugged or screwed onto the device. The integral component is preferably disposable such that the treatment head tip and the cavity may be replaced after a certain number of uses, for instance, when the abrasiveness of the abrasive surface decreases by skin cell clogging the abrasive material. A further advantage of such a disposable component may be improved hygiene. Furthermore, it is conceivable that the multiple integral components of such type are provided together with the device each having different first and second abrasive surfaces with different abrasion characteristics.

According to the present invention, the cavity has an essentially dome-like shape with an annular surrounding, wherein the treatment head tip is formed by the annular surrounding and the inner walls are formed by an inner surface of said dome-like shape.

The treatment head tip is formed around the edge of the cavity, preferably as an annular surface that stretches around the cavity and on which the first abrasive surface is arranged. The first abrasive surface is thus preferably arranged on a planar plane on one side of the device, whereas the second abrasive surface is arranged on the inner walls of the dome-like shape extending into the device. Thus, when the device is placed for treatments with the treatment head tip on top of the user's skin, only the first abrasive surface is in contact with the skin. Only if an under-pressure is applied the skin will be drawn into the cavity, forming thereby a skin dome that evenly fits into the dome-like shape. The even dome-like shape thus allows that the drawn-in skin contacts the second abrasive surface evenly. Hence, in embodiments the first abrasive surface may substantially perimetrically surround the second abrasive surface.

Furthermore, since the treatment head tip is arranged annularly around the cavity, the first abrasive surface always contacts the skin first regardless in which direction the device is moved on the user's skin. Additionally, the treatment of a part of the skin always ends with a treatment by the first abrasive surface allowing a relaxation of the skin if the second abrasive surface has a higher abrasion level or even perforates through the stratum corneum of the user's skin.

According to a further embodiment of the present invention, the second abrasive surface is divided into multiple adjacent sections having each a different abrasive characteristic.

In this embodiment the second abrasive surface comprises multiple sections of different abrasive characteristic. For that, different areas of the second abrasive surface may be covered with different abrasive material such as sharp particles with varying particle sharpness. Alternatively, all sections are covered with the same abrasive material, but have been treated with different or different amounts of filling material that reduces the sharpness of the abrasive material. Preferably, the sections are arranged in such a way that by applying different levels of under-pressure to the cavity only certain sections are able to get in contact with the user's skin. This way, a selected treatment of the skin with different abrasion levels in a single treatment is feasible, wherein the abrasion level may be easily controlled by the level of under-pressure applied. It shall be noted that the term "adjacent sections" shall not necessarily imply that theses sections are arranged directly adjacent/neighboring to one another, even though this is preferred. The "adjacent sections" shall also include an adjacent arrangement of the sections, wherein a (small) gap occurs in between them. It is also possible that the roughness of the second abrasive surface increases or decreases continuously with decreasing distance to the opening axis. In the last mentioned embodiment the adjacent sections are thus infinitesimal small.

According to a further embodiment of the present invention, the second abrasive surface comprises sharp objects protruding from said second abrasive surface.

The sharp objects may be micro needles, grown crystals or pointy sharp particles that are arranged alone or together with other abrasive material on the second abrasive surface. When sufficient under-pressure is applied to the cavity such that the second abrasive surface and the sharp objects get in contact with the user's skin, the sharp objects may penetrate all the way through the stratum corneum in addition to the abrasion of the top layers of the skin. This way, topicals or lotions that are applied onto the skin before or after the microdermabrasion treatment may penetrate through this perforation into the deeper layer of the epidermis, hence further improving the effectiveness of the topical treatment. Preferably, the sharp objects are arranged close to the opening such that a high under-pressure has to be applied for the sharp objects to become effective.

In an even more preferred embodiment said sharp objects protrude from said surface and have a length of between 5 to 25 µm.

The sharp objects may comprise abrasive structures, such as particulate material, having mean dimensions (such as length) in the range of 1-1000 µm, such as 2-300 µm, like 5-80 µm, such as 5-25 µm, or 120-200 µm. The microscopic abrasive may for instance be selected from the group consisting of silicon carbide structures, such as silicon carbide particles, and metal nitride structures, such as metal nitride particles. Alternatively or additionally, the abrasive structures may for instance be selected from the group consisting of metal oxide structures, such as aluminum oxide particles and aluminum oxide structures. Further options of abrasive structures may for instance be selected from the group consisting of diamond structures, boron nitride structures, silicon carbide structures, glass beads, steel grit structures, other metal grit structures, zirconium oxide structures, and quartz structures. Combinations of different kind of abrasive structures, both in chemical composition and/or dimensions, may also be applied.

This particular dimension of the sharp objects has the advantage that only the stratum corneum may be perforated by the sharp objects, whereas deeper layers of the epidermis such as the stratum granulosum, stratum spinosum, or the basal layer remains unaffected. This way, an accidental injury of the user's skin can advantageously be avoid.

According to a further embodiment of the present invention, the device further comprises a vacuum source which is connected to the opening.

The vacuum source connected to the opening may be a standalone unit which is separate from the device and connectable thereto. However, the vacuum source may also be directly integrated into the device. For the latter, the device may further comprise a power supply, preferably in form of a rechargeable battery that even more preferably is rechargeable in a wirelessly manner, for instance, via induction. In case of a standalone vacuum source a tube may be provided that connects the vacuum source with the opening to provide a suction path.

The vacuum source is configured to provide an under-pressure within the cavity such that skin is drawn into the cavity towards the opening when the treatment head tip is placed onto the user's skin. Within the cavity the skin is exposed to the second abrasive surface. The vacuum source may provide at least two different under-pressure levels. A first under-pressure level may be defined by a turned-off vacuum source, whereas a second under-pressure level may be defined by the vacuum source running at maximum power. Preferably, the vacuum source is configured to provide further under-pressure levels, wherein each under-pressure level may lead to different abrasion intensity in combination with the second abrasive surface. It is also conceivable that the vacuum source may provide an infinitely adjustable under-pressure within the cavity.

In addition, the under-pressure level may alternatively be determined by a valve system arranged along the suction path capable of providing a deliberate leakage such that different under-pressure levels are achievable with a constant vacuum source. Such a valve system could be arranged within the treatment head and may be configured as a pneumatic oscillator providing an alternating under-pressure.

According to a further embodiment of the present invention, the device comprises a controller which is configured to control the vacuum source to apply an alternating under-pressure within the cavity.

The controller adjusts the under-pressure within the cavity automatically, preferably by controlling the power supply of the vacuum source. At least two different under-pressure levels can be set by the controller. At a first under-pressure level the skin is only exposed to the first abrasive surface, whereas at a second level the skin is additionally exposed to the second abrasive surface. The controller alternates between these at least two levels such that a fractionated abrasion is achieved that allows locally a very intensive treatment.

Hence, in embodiments the controller is configured to control the vacuum source, especially to control the under-pressure within the cavity (when the device is applied to the skin. In yet further specific embodiments, the controller may be configured to alternate between these at least two levels such that a fractionated abrasion is achieved that allows locally a very intensive treatment. Further, the device may include a user interface. Especially, the user interface may be configured to allow a user to control the vacuum source. For instance, the user interface may be used to control the level of underpressure of the first under-pressure level and/or the second under-pressure level. The controller may be configured to control the vacuum source at a plurality of underpressures, such as e.g. a stepwise controllable underpressure.

Especially, the device may be configured to provide a negative pressure ("underpressure") in the range of 5-80 kPa, such as especially 15-60 kPa, such as in the range of 20-40 kPa. This may especially imply that when the skin is in contact with the treatment head, and closes off the cavity, the device is able to provide a pressure which is in the range of 15-60 kPa lower than atmospheric pressure. Hence, the term underpressure may especially indicate that when the skin is in contact with the treatment head, the skin may be sucked at least partly into the cavity due to the suction of the vacuum source, leading to an underpressure in the cavity relative to ambient pressure.

Especially, the channel opening (with its channel rim) is configured such that a suitable vacuum area is obtained. Especially, the channel rim has (or provides) a vacuum area in the range of 10-400 mm², such as 10-200 mm², like 45-75 mm², such as especially 50-75 mm². This vacuum area is especially the area enclosed by the channel rim. It is especially also the (cross-sectional) area of the inlet zone.

According to a further embodiment of the present invention, the inner wall comprises a flexible section.

In this embodiment the inner walls are at least partially formed by a flexible section. The flexible section is able to move inwardly into the cavity upon generation of an under-pressure to the cavity. During movement of the treatment head tip over the skin surface, when an under-pressure is applied, the flexible section of the inner wall is forced inwardly towards the skin of the user thus enhancing the penetration of the abrasive particles into the skin. The abrasive particles penetrate the stratum corneum and thus enable a better penetration of a topical into the deeper layers of the skin.

According to a further embodiment of the present invention the second abrasive surface is arranged on said flexible section.

In this embodiment the second abrasive surface is arranged on the flexible section of the inner wall. When the flexible section moves inwardly towards the skin of the user due to an under-pressure supplied to the cavity, the second abrasive surface gets in contact with the skin. The flexible section can be configured such that they only move inwardly when a certain under-pressure level is set which allows a very precise control over the application of the second abrasive surface. Furthermore, this assembly allows the second abrasive surface to be arranged at a distance far enough from the skin of the user during periods where no under-pressure is applied, such that an accidental and unwanted abrasion by the second surface may be ruled out. At the same time, when an under-pressure is applied to the cavity, the flexible section may move quickly and forcefully against the skin such that the desired abrasion by the second abrasive surface is achieved.

In the present invention, the treatment head is especially stationary. Hence, the device may especially in embodiments not be configured to move the treatment head relative to the remainder of the divice (such as a rotational movement about a opening axis).

### BRIEF DESCRIPTION OF THE DRAWINGS

These and other aspects of the invention will be apparent from and elucidated with reference to the embodiments described hereinafter. In the following drawings
Fig. 1 shows a first embodiment of a device for microdermabrasive skin treatment according to the present invention in a perspective view,
Fig. 2 shows a second embodiment of a device for microdermabrasive skin treatment according to the present invention,
Fig. 3 shows an exemplary configuration of an abrasive surface,
Fig. 4 shows a third embodiment of a device for microdermabrasive skin treatment according to the present invention,
Fig. 5 shows a fourth embodiment of a device for microdermabrasive skin treatment according to the present invention,
Fig. 6 schematically shows patterns of application of a device according to the present invention, and
Fig. 7 schematically shows a skin of the user treated by a device according to the present invention.

### DETAILED DESCRIPTION OF EMBODIMENTS

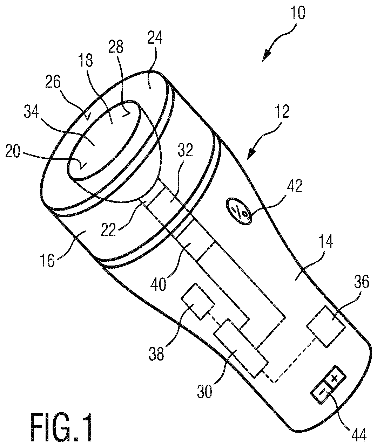

Fig. 1 shows a perspective view of a first embodiment of the device for microdermabrasive treatment of the skin of a user according to the present invention. The device is denoted in its entirety with reference number 10.

The device comprises an elongate body 12 with a handle 14 for holding the device 10 during the microdermabrasive treatment and a treatment head 16 which is attached to the handle 14. The treatment head 16 is configured to abrade top layers of the skin of a user when moved over the top layer of the user's skin. The treatment head 16 comprises a cavity 18 which forms with its inner walls 20 preferably an even pit within an essentially planar front surface of the treatment head 16. Furthermore, an opening 22 in the form of a through hole into the device is embedded at the bottom of the pit-like cavity 18. The treatment head 16 further comprises a treatment head tip 24 which is arranged annular around the edge of the cavity 18. The treatment head tip 24 is the part of the device which will be placed onto the skin of the user to start the microdermabrasive treatment.

The treatment head tip 24 is preferably designed as a flat ring forming a rim around the cavity 18. On the rim of the treatment head tip 24 a first abrasive surface 26 is arranged facing the skin of the user when the treatment head tip 24 is placed thereupon. A second abrasive surface 28 is arranged within the cavity 18 covering at least a part of the inner walls 20. The first abrasive surface 26 is essentially arranged on a planar surface, whereas the second abrasive surface 28 is arranged on a bended surface defined by the concave shape of the cavity 18. Both abrasive surfaces 26, 28 are coated with abrasive material, for instance, hard minerals or synthetic stones, and are configured to abrade layers of skin, in particular dead skin cells, when pressed onto and moved over the skin. Preferably, the treatment head tip 24 and the cavity 18 form an integral component that is either releasably or fixedly connected to the handle 14 of the treatment head 16.

The opening 22 within the cavity 16 is configured to be connected to a vacuum source 30 which can be any device configured to remove a gas, in particular air, from a sealed volume in order to leave behind a partial vacuum. In this preferred embodiment the vacuum source 30 is integrated into the body 12 of the device 10 as denoted here by the dashed block. In other embodiments a vacuum source may be provided separately as a standalone unit which is connectable to an inlet of the device. The integrated vacuum source 30, or alternatively the inlet, are connected to the opening 22 via a suction path 32 to extract air from within the cavity 18 and to generate an under-pressure within the volume 34 formed by the cavity 18, the treatment head tip 24 and the skin of the user, when the treatment head tip 24 is placed thereupon.

The device 10 may further comprise a power supply 36, either externally provided or integrated in form of a battery pack, a controller 38 for controlling the vacuum source 30, as well as filters or removable reservoirs 40 that are arranged along the suction path 32 to collect abraded skin particles which are sucked into the device through the opening 22. The controller 38, the filters and reservoirs 40 may as well be arranged external to the device such that the device essentially only comprises the handle 14, the treatment head 16, and the treatment head tip 24 with the cavity 18 formed as an integral component. Preferred, however, is a fully integrated device residing in a waterproof housing with a rechargeable power supply and removable components for the expendable materials.

Furthermore, the device 10 may comprise a user interface illustrated here by a power-on button 42 and a control switch 44 arranged on the handle 14. With the power-on button 42 the vacuum source 30 may be activated to generate an under-pressure within the cavity 18, wherein the control switch 44 may be used to set different under-pressure levels. The control switch 44 is preferably connected to the controller 38 which is configured to control volume flow rate of the vacuum source 30, for instance, by regulating the power supply 36.

One of the central points of the device 10 is the placement and the configuration of the first and second abrasive surface 26, 28. By arranging the second abrasive surface 28 inside the cavity 16 and essentially transverse to the first abrasive surface 26, both surfaces 26, 28 may generally not get in contact with the user's skin at the same time. In fact, the second abrasive surface 28 may only get in contact with the user's skin when an under-pressure is applied to the cavity 18. Furthermore, the first and second abrasive surface 26, 28 are configured with different roughness to provide different abrasion levels. In other words, the amount of skin abraded and the way, how the user's skin is abraded, by the first and second abrasive surface 26, 28 varies between the first and second abrasive surface 26, 28.

A microdermabrasive treatment with the claimed device, hence, envisages at least partially an abrasion with different abrasion levels. At first the skin is abraded by the first abrasive surface 26, when the device 10 is moved across the surface of the skin of the user, and additionally by the second abrasive surface 28 which has a different, preferably a more intense, abrasion ability. Applying the second abrasive surface 28 is thereby optionally and can be controlled over the level of under-pressure applied to the user's skin.

The function of the under-pressure is manifold. On the one hand, the under-pressure assists in collecting loosened particles within the device 10 in a filter or reservoir 40 by creating airflow above the skin. The airflow may be created by tiny holes in the treatment head tip or by lifting the device briefly from the skin of the user. On the other hand, the under-pressure provides a vacuum massage of the user's skin to enhance the blood circulation within the skin. Additionally, according to the present invention the under-pressure also controls the abrasion level by drawing skin into the cavity 18, and forcing it against the second abrasive surface 28 which is arranged therein. In other words, if no under-pressure is applied and the treatment head tip 24 is moved over the user's skin, only the first abrasive surface 26 is in contact with the skin and abrades the top layer. Once an under-pressure is applied, the skin is additionally treated by the second abrasive surface 28 providing an additional, preferably more intense, abrasion of the top layers.

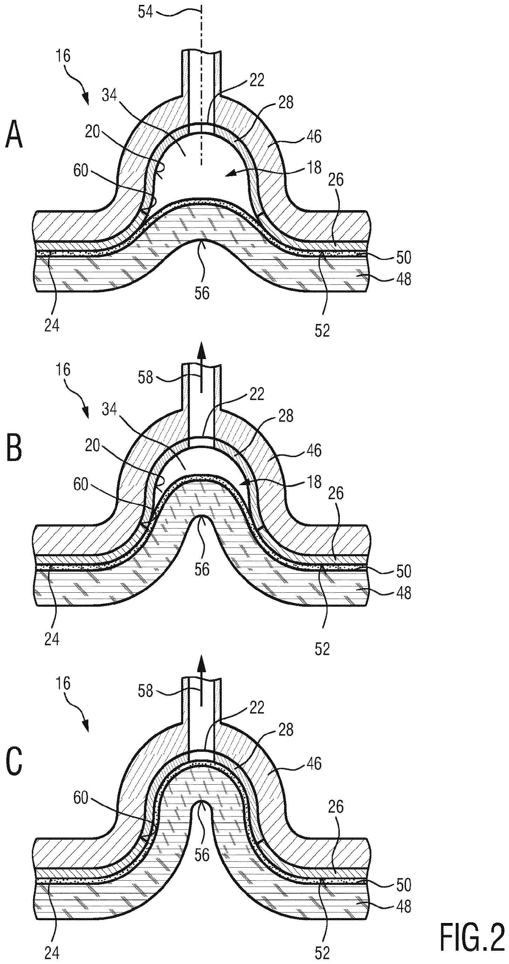

Fig. 2 shows a second embodiment of a device 10 for microdermabrasive treatment in a cross-sectional schematic view. The device 10 is essentially similar to the embodiment shown in Fig. 1 besides a different configuration of the treatment head 16 which is shown here for simplification alone without the rest of the body. Furthermore, the treatment head 16 is shown here at three different stages A, B, C of the microdermabrasive treatment.

The cavity 18 and the treatment head tip 24 are combined into an integral component 46 providing a continuous surface on which the first and second abrasive surface 26, 28 are adjacently arranged. The treatment head tip 24 comprises a front surface 52 which is at least partially arranged parallel to the surface of the skin of the user and comprises the first abrasive surface 26. The skin is denoted here with reference numeral 48 and the top layer of the skin 48, the stratum corneum, is denoted with reference numeral 50.

The front surface 52 gradually transitions in a smooth curve into the inner walls 20 of the cavity 18, wherein the cavity 18 has a pit-like shape with the opening 22 arranged on the bottom of said pit. The opening 22 defines an opening axis 54 and is preferably arranged in the center of the cavity 18, such that the inner wall 20 and the front surface 52 of the treatment head tip 24 are arranged concentrically about the opening axis 54. When the treatment head tip 24 is placed onto the skin 48, the cavity 18 is arranged like a bell on top of the skin 48 annually sealed by the treatment head tip 24. To the sealed volume 34 formed by the skin 48 and the cavity 18 an under-pressure can be applied through the opening 22 by means of the vacuum source 30. The under-pressure in the sealed volume 34 causes the parts of the elastic skin 48 located underneath the cavity 18 to be drawn towards the direction of opening 22 forming a skin dome denoted here with reference numeral 56. The skin dome 56 increases in size, the more under-pressure is applied (i.e. the higher the vacuum) in the sealed volume 34.

In the top drawing (A) of Fig. 2 no under-pressure is applied to the volume 34 and the skin 48 only forms a small skin dome 56 mainly caused by the pressure with which the treatment head tip 24 is pressed onto the skin. In this constellation, the skin 48 is only in contact with the first abrasive surface 26, yet not with the second abrasive surface 28.

In the middle drawing (B) of Fig. 2 some under-pressure is applied. The under-pressure level is indicated here by the thickness of the arrow 58. A greater skin dome 56 is formed and the skin 48 is additionally exposed to at least some parts of the second abrasive surface 28.

In the bottom drawing (C) of Fig. 2 a maximum under-pressure is applied forcing as much skin 48 into the cavity 18 as possible. Thereby, the skin 48 is contacted by the first abrasive surface 26 and the entire second abrasive surface 28 for the highest abrasion level.

The central point of the second embodiment is the configuration of the first and second abrasive surface 26, 28 which are provided here as a continuous, yet inhomogeneous layer. In particular, the roughness of the second abrasive surface 28 increases, starting preferably from a low abrasiveness at the edge 60 between the first and second abrasive surface 28, up to a high abrasiveness towards the opening 22. In other words, the first and the second abrasive surface 26, 28 provide a gradient in abrasiveness that increases in the direction of the opening 22. Closer to the opening 22 a roughness of the abrasive surface is higher than at the edge to the treatment head tip 24. This way, in this embodiment a variation in the under-pressure level 58 in the cavity 18 may be used in combination with varying abrasive levels of the second abrasive surface 28 to allow a fine-grained control of the abrasion such that locally high abrasion levels may be applied.

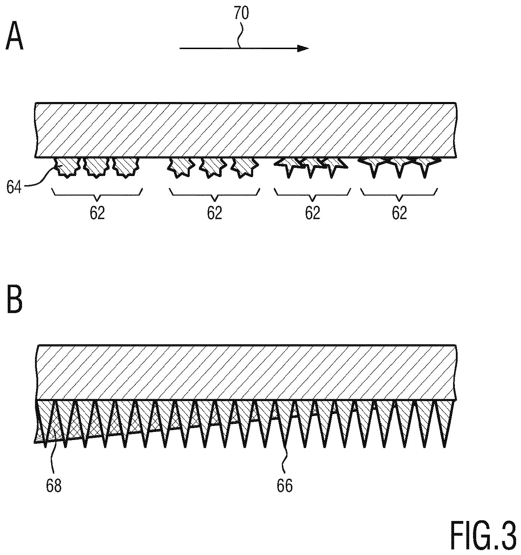

A gradient in abrasion ability of the abrasive surface as shown in Fig. 2 may be achieved in multiple ways. Fig. 3 shows two exemplary configurations A and B of abrasive surfaces with a gradient in abrasion ability. The first example A of Fig. 3 shows an abrasive surface having different sections 62 each coated with particles 64, for instance hard minerals or synthetic stones, of different sharpness. To achieve an increasing abrasion level each adjoining section comprises particles with increased sharpness.

The second example B of Fig. 3 shows an abrasive surface which is formed from a homogenous abrasive material, for instance, by a layer of similar sharp particles. Alternatively, the abrasive surface comprises pointy micro needles 66 which protrude from the surface and are arranged in parallel to each other to form a layer of homogenous abrasiveness. The homogenous layer is partially filled with a filing material 68 to reduce the sharpness of the homogenous layer in certain areas. The filling material fills up the sharp edges of the particles 64 or the gaps between the micro needles 66, thereby reducing their sharpness. Preferably, the amount of filling material 68 applied to the abrasive surface is gradually reduced from on side of the abrasive surface to an opposite side. This way, a continuous increase of abrasiveness of the surface in one direction is achieved as denoted here by reference numeral 70. On the left-hand side the micro needles 66 are almost fully covered by the filling material 68 such that only low abrasion ability is provided, whereas on the right-hand side the micro needles 66 are basically not covered with filling material 68 at all, and the micro needles 66 thus provide their original sharpness.

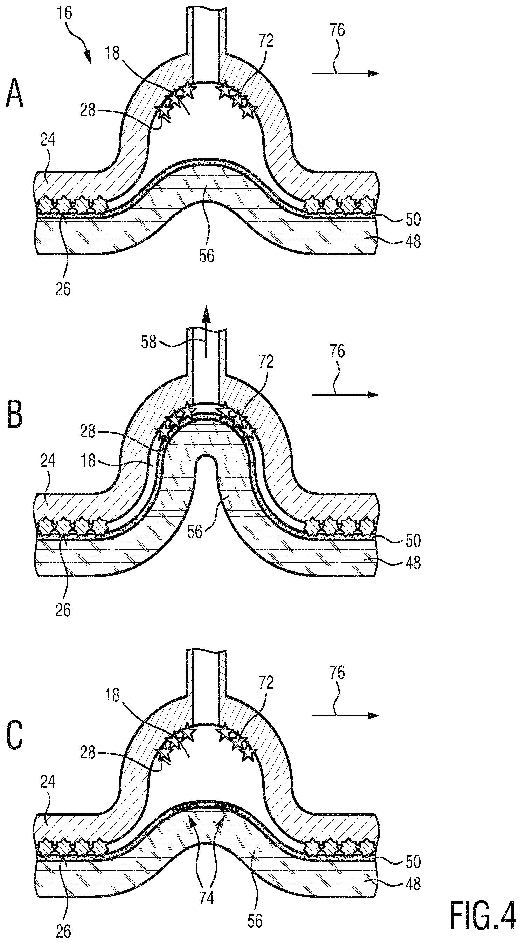

Fig. 4 shows a third embodiment of a device for microdermabrasive treatment in a cross-sectional schematic view. The device is essentially similar to the embodiments shown in Fig. 1 and 2, yet again with a different configuration of the treatment head 16. The rest of the arrangement stays the same as shown and described according to the first and second embodiment.

Fig. 4 shows a treatment head 16 with different abrasive surfaces 26, 28 arranged on the treatment head 24 and the inner walls 20 of the cavity 18. In particular, the second abrasive surface 28 comprises in this embodiment sharp objects 72 which protrude from the surface of the inner walls 20 like tiny spearheads. The sharp objects 72 are for example sharp pointy particles, grown crystals or tiny micro needles and are configured to cut all the way through the stratum corneum 50 when pressed against the skin 48. In other words, the sharp objects 72 perforate the stratum corneum 50 locally creating small perforations 74 in the stratum corneum 50.

Fig. 4 shows similar to Fig. 2 three different stages A, B, C of the microdermabrasive treatment. The function of the treatment head 16 remains essentially the same. Once an under-pressure 58 is applied to the cavity 18 (B) a skin dome 56 is formed, drawn into the cavity 18, and thereby pressed against the second abrasive surface 28 comprising here the sharp objects 72. The sharp objects 72 cut into the skin 48 and leave small perforations 74 in the stratum corneum 50 behind to enhance the penetration of topicals into deeper layers of the skin 48. Once the under-pressure 58 is reduced (C) the skin dome 56 is released and the skin 48 falls back into its original position. Meanwhile, the treatment head 16 is moved further along the surface of the skin 48 as denoted here with reference numeral 76, removing thereby constantly top layers of the skin with the help of the first abrasive surface 26 arranged on the front surface 52 of the treatment head tip 24. At a different part of the skin 48 the procedure may be repeated, wherein the under-pressure 58 is increased again to cut further perforations 74 into the stratum corneum 50 at a different position.

The amount and length of the perforations 74 and thereby the coverage is determined by amount, size and sharpness of the sharp objects 72, the duration of an under-pressure burst, and the movement speed 76.

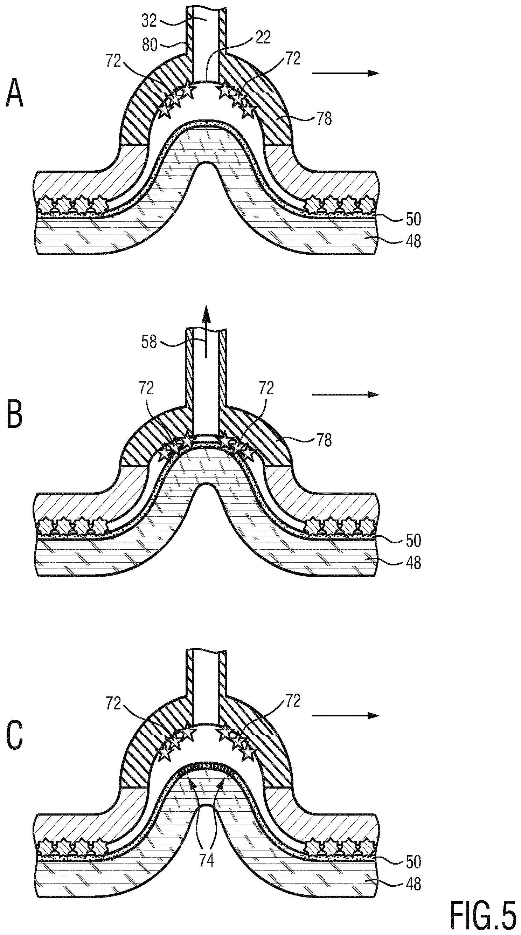

Fig. 5 shows a fourth embodiment of a device for microdermabrasive treatment in a cross-sectional schematic view. The device is essentially similar to the preceding embodiments, yet again with a different configuration of the treatment head 16, whereas the rest of the arrangement stays essentially the same as shown and described before.

Fig. 5 shows a treatment head 16, wherein the inner walls 20 of the cavity 18 comprise a flexible section 78. Preferably, the flexible section 78 comprises a flexible membrane that is arranged concentrically around the opening 22, wherein the opening 22 may itself be embedded into the flexible section 78. On the surface of the flexible section 78 the second abrasive surface 28 is arranged, which in this particular embodiment comprises sharp objects 72 as described in detail with reference to Fig. 4. The sharp objects 72 are configured to penetrate through the stratum corneum 50 when pressed against the skin 48.

As before, in Fig. 5 three different stages A, B, C of the microdermabrasive treatment are shown. When no under-pressure 58 is applied to the cavity 18 (A), the flexible membrane 78 stays in its initial bell-shaped form. In that shape the flexible membrane 78 and the second abrasive surface 28, which is arranged thereupon, do not get in contact with the skin 48. Preferably, the initial shape of the flexible membrane as well as the dimension of the cavity 18 is configured such that it is physically impossible for the skin 48 to touch the second abrasive surface 28 when the treatment head tip 24 is normally placed onto the skin 48.

Once an under-pressure 58 is applied to the cavity 18 (B), the flexible membrane 78 bends inwardly, pressing the second abrasive surface 28 against the skin 48. Thereby, the stratum corneum 50 is perforated in a similar manner as explained before with reference to Fig. 4. After the under-pressure is reduced (C), the flexible membrane 78 snaps back into its initial shape, thereby withdrawing the sharp objects 72 from the skin 48.

The flexible membrane 78 may be configured to additionally facilitate the perforation. For that, the flexible membrane 78 comprises besides the initial shape a further steady state in which the membrane is drawn when an under-pressure 58 is applied. The perforation is thereby advantageously enhanced by the inner tension force of the membrane 78.

It comes without saying that according to the fourth embodiment the opening 22 may move together with the flexible section 78 when an under-pressure 58 is applied. The suction path 32 may therefore comprise of a flexible tube 80 connected to the opening 22 that moves along with opening 22 if necessary.

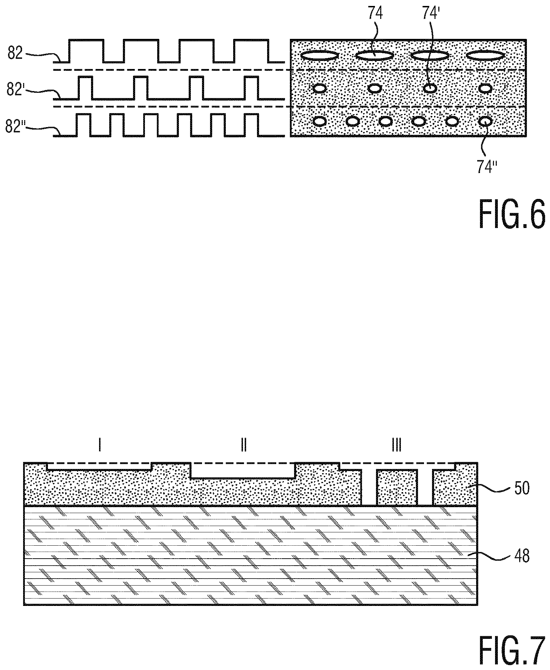

With reference to Fig. 6 and 7 a preferred skin treatment using any of the above embodiments is described. This is achieved by applying under-pressure to the cavity 18 in an alternating manner, wherein the duty cycle is preferably adjustable by the user.

Fig. 6 shows schematically patterns of application of a device according to the present invention. In particular, Fig. 6 shows on the left hand side three different periods and duty cycles 82 of a vacuum source attached to the device and on the right hand side the resulting perforation patterns created thereby.

On top, a high duty cycle 82 with long alternating periods of applied under-pressure is depicted. Such duty cycle and period leads to a high coverage with long perforations 74 in the skin of the user. In the middle, a short duty cycle 82' with short alternating periods of applied under-pressure is depicted. Such duty cycle and period leads to a low coverage with only short perforations 74' in the skin of the user. Finally, on the bottom a high duty cycle 82" with short alternating periods is depicted. Such duty cycle and period leads to a high coverage with short perforations 74" in the skin of the user.

It goes without saying that the coverage also depends on the speed with which the device is moved over the user's skin. In the above examples an equal and constant speed is assumed.

Fig. 7 shows schematically skin of the user treated by a device according to the present invention. In particular, Fig. 7 shows three different effects (I, II, II) achieved by a microdermabrasive treatment with a device according to the present invention.

The first effect I is a light abrasion of the top layer of the skin. Hereby, only a small amount of the stratum corneum 50 is abraded. Such abrasion may be achieved by the first abrasive surface 26 arranged on the treatment head tip 24 of the device. The second effect II is a middle abrasion of the stratum corneum 50. This my be achieved by additionally applying a higher abrasiveness of the second abrasive surface 28 to the skin 48, and thereby abrading more layers of the stratum corneum 50. Finally, effect III shows a light abrasion, which is achieved by the first abrasive surface 26 combined with a very short, yet deep perforation of the all the way through the stratum corneum 50 achieved by intensely applying the second abrasive surface 28 to the skin.

All four embodiments described within the corresponding figures share the same inventive concept of providing a microdermabrasive skin treatment with different abrasion levels with a single device in a single session. For that, the device comprises a first and a second abrasive surface 26, 28, wherein the roughness of the first abrasive surface 26 differs from the roughness of the second abrasive surface 28. The four embodiments only differ in the configuration of the treatment head 16 which comprises the first and second abrasive surface 26, 28.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. The term "comprising" may in an embodiment refer to "consisting of' but may in another embodiment also refer to "containing at least the defined species and optionally one or more other species". The term "comprise" includes also embodiments wherein the term "comprises" means "consists of'. The term "and/or" especially relates to one or more of the items mentioned before and after "and/or". For instance, a phrase "item 1 and/or item 2" and similar phrases may relate to one or more of item 1 and item 2. A single element or other unit may fulfill the functions of several items recited in the claims. The mere fact that certain measures are recited in mutually different dependent claims does not indicate that a combination of these measures cannot be used to advantage.

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The invention may be implemented by means of hardware comprising several distinct elements, and by means of a suitably programmed computer. In the device claim enumerating several means, several of these means may be embodied by one and the same item of hardware.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

Any reference signs in the claims should not be construed as limiting the scope.

## Claims

1. Device for microdermabrasive treatment comprising:
a treatment head (16) having a cavity (18) with inner walls (20), an opening (22) in said inner walls, and a treatment head tip (24) for placing the treatment head (16) on a skin (48) of the user,
wherein the treatment head tip (24) comprises a first abrasive surface (26) and the inner walls (20) comprise a second abrasive surface (28),
wherein the opening (22) is configured to be connected to a vacuum source (30) for applying an under-pressure (58) within the cavity (18), when the treatment head tip (24) is placed on the skin (48) of the user,
wherein the first abrasive surface (26) comprises a different roughness than the second abrasive surface (28),
wherein the device is configured to have the second abrasive surface (28) only contact the skin (48) of the user when the under-pressure (58) is applied to the cavity (18); and
wherein the cavity (18) has an essentially dome-like shape with an annular surrounding, and wherein the treatment head tip (24) is formed by the annular surrounding, and the inner walls (20) are formed by an inner surface of said dome-like shape.

2. Device according to claim 1, wherein a roughness of the second abrasive surface (28) is higher than a roughness of the first abrasive surface (26).

3. Device according to any of the claims 1-2, wherein the treatment head tip (24) is arranged on a first side of the cavity (18) and the opening (22) is arranged on an opposite side of the cavity (18), and wherein the roughness of the second abrasive surface (28) increases towards the opening (22).

4. Device according to any of the claims 1-3, wherein the first abrasive surface (26) is arranged transverse to the second abrasive surface (28).

5. Device according to any of the claims 1-4, wherein the opening (22) defines an opening axis (54), and wherein the first abrasive surface (26) and the second abrasive surface (28) are arranged concentrically around said opening axis (54).

6. Device according to claim 5, wherein the second abrasive surface (28) is arranged closer to said opening axis (54) than the first abrasive surface (26).

7. Device according to any of the claims 1-6, wherein the treatment head tip (24) and the cavity (18) are arranged to form a single integral component (46).

8. Device according to any of the claims 1-7, wherein the second abrasive surface (28) is divided into multiple adjacent sections (62) having each a different abrasive characteristic.

9. Device according to any of the claims 1-8, wherein the second abrasive surface comprises (28) sharp objects (72) protruding from said second abrasive surface (28).

10. Device according to claim 9, wherein said sharp objects (72) protrude from said surface and have a length of between 5 to 25 µm.

11. Device according to any of the claims 1-10, further comprising a vacuum source (30) which is connected to the opening (22).

12. Device according to claim 11, further comprising a controller (38) which is configured to control the vacuum source (30) to apply an alternating under-pressure within the cavity (18).

13. Device according to any of the claims 1-12, wherein the inner wall (22) comprises a flexible section (78).

14. Device according to claim 13, wherein the second abrasive surface (28) is arranged on said flexible section (78).

## Patentansprüche

1. Vorrichtung zur Behandlung mittels Mikroabtragung von Hautgewebe, umfassend:
einen Behandlungskopf (16) mit einem Hohlraum (18) mit Innenwänden (20), einer Öffnung (22) in den Innenwänden, und einer Behandlungskopfspitze (24) zum Platzieren des Behandlungskopfs (16) auf einer Haut (48) des Anwenders,
wobei die Behandlungskopfspitze (24) eine erste Abtragungsfläche (26) umfasst und die Innenwände (20) eine zweite Abtragungsfläche (28) umfassen,
wobei die Öffnung (22) konfiguriert ist, mit einer Vakuumquelle (30) verbunden zu werden, um einen Unterdruck (58) innerhalb des Hohlraums (18) anzuwenden, wenn die Behandlungskopfspitze (24) auf der Haut (48) des Anwenders platziert wird,
wobei die erste Abtragungsfläche (26) eine andere Rauheit als die zweite Abtragungsfläche (28) umfasst,
wobei die Vorrichtung so konfiguriert ist, dass die zweite Abtragungsfläche (28) die Haut (48) des Anwenders nur kontaktiert, wenn Unterdruck (58) auf den Hohlraum (18) angewendet wird; und
wobei der Hohlraum (18) eine im Wesentlichen kuppelartige Gestalt mit einer ringförmigen Umgebung aufweist, und wobei die Behandlungskopfspitze (24) mittels der ringförmigen Umgebung gebildet wird, und die Innenwände (20) mittels einer Innenfläche der kuppelartigen Gestalt gebildet werden.

2. Vorrichtung nach Anspruch 1, wobei eine Rauheit der zweiten Abtragungsfläche (28) höher ist als eine Rauheit der ersten Abtragungsfläche (26).

3. Vorrichtung nach einem der Ansprüche 1-2, wobei die Behandlungskopfspitze (24) an einer ersten Seite des Hohlraums (18) angeordnet ist und die Öffnung (22) an einer gegenüberliegenden Seite des Hohlraums (18) angeordnet ist, und wobei die Rauheit der zweiten Abtragungsfläche (28) in Richtung der Öffnung (22) zunimmt.

4. Vorrichtung nach einem der Ansprüche 1-3, wobei die erste Abtragungsfläche (26) quer zu der zweiten Abtragungsfläche (28) angeordnet ist.

5. Vorrichtung nach einem der Ansprüche 1-4, wobei die Öffnung (22) eine Öffnungsachse (54) definiert, und wobei die erste Abtragungsfläche (26) und die zweite Abtragungsfläche (28) konzentrisch rund um die Öffnungsachse (54) angeordnet sind.

6. Vorrichtung nach Anspruch 5, wobei die zweite Abtragungsfläche (28) näher zu der Öffnungsachse (54) angeordnet ist als die erste Abtragungsfläche (26).

7. Vorrichtung nach einem der Ansprüche 1-6, wobei die Behandlungskopfspitze (24) und der Hohlraum (18) angeordnet sind, um eine einzelne, einstückige Komponente (46) zu bilden.

8. Vorrichtung nach einem der Ansprüche 1-7, wobei die zweite Abtragungsfläche (28) in mehrere benachbarte Abschnitte (62) mit jeweils einer anderen Abtragungscharakteristik unterteilt ist.

9. Vorrichtung nach einem der Ansprüche 1-8, wobei die zweite Abtragungsfläche (28) scharfe Objekte (72) umfasst, die aus der zweiten Abtragungsfläche (28) herausragen.

10. Vorrichtung nach Anspruch 9, wobei die scharfen Objekte (72) aus der Fläche herausragen und eine Länge von zwischen 5 bis 25 µm aufweisen.

11. Vorrichtung nach einem der Ansprüche 1-10, weiter eine Vakuumquelle (30) umfassend, welche mit der Öffnung (22) verbunden ist.

12. Vorrichtung nach Anspruch 11, weiter eine Steuerung (38) umfassend, welche konfiguriert ist, die Vakuumquelle (30) zu steuern, um einen alternierenden Unterdruck innerhalb des Hohlraums (18) anzuwenden.

13. Vorrichtung nach einem der Ansprüche 1-12, wobei die Innenwand (22) einen flexiblen Abschnitt (78) umfasst.

14. Vorrichtung nach Anspruch 13, wobei die zweite Abtragungsfläche (28) auf dem flexiblen Abschnitt (78) angeordnet ist.

## Revendications

1. Dispositif de traitement microdermabrasif comprenant :
une tête de traitement (16) comportant une cavité (18) ayant des parois intérieures (20), une ouverture (22) dans lesdites parois intérieures, et une pointe de tête de traitement (24) pour placer la tête de traitement (16) sur une peau (48) de l'utilisateur,
dans lequel la pointe de tête de traitement (24) comprend une première surface abrasive (26) et les parois intérieures (20) comprennent une seconde surface abrasive (28),
dans lequel l'ouverture (22) est configurée pour être connectée à une source de vide (30) pour appliquer une sous-pression (58) à l'intérieur de la cavité (18), lorsque la pointe de tête de traitement (24) est placée sur la peau (48) de l'utilisateur,
dans lequel la première surface abrasive (26) présente une rugosité différente de celle de la seconde surface abrasive (28),
dans lequel le dispositif est configuré pour n'avoir que la seconde surface abrasive (28) en contact avec la peau (48) de l'utilisateur lorsque la sous-pression (58) est appliquée à la cavité (18) ; et
dans lequel la cavité (18) a une forme essentiellement de type dôme avec une enveloppe annulaire, et dans lequel la pointe de tête de traitement (24) est formée par l'enveloppe annulaire, et les parois intérieures (20) sont formées par une surface intérieure de ladite forme de type dôme.

2. Dispositif selon la revendication 1, dans lequel une rugosité de la seconde surface abrasive (28) est supérieure à une rugosité de la première surface abrasive (26).

3. Dispositif selon l'une quelconque des revendications 1 et 2, dans lequel la pointe de tête de traitement (24) est agencée sur un premier côté de la cavité (18) et l'ouverture (22) est agencée sur un côté opposé de la cavité (18), et dans lequel la rugosité de la seconde surface abrasive (28) augmente en direction de l'ouverture (22).

4. Dispositif selon l'une quelconque des revendications 1 à 3, dans lequel la première surface abrasive (26) est agencée transversalement à la seconde surface abrasive (28).

5. Dispositif selon l'une quelconque des revendications 1 à 4, dans lequel l'ouverture (22) définit un axe d'ouverture (54), et dans lequel la première surface abrasive (26) et la seconde surface abrasive (28) sont disposées de manière concentrique autour dudit axe d'ouverture (54).

6. Dispositif selon la revendication 5, dans lequel la seconde surface abrasive (28) est agencée plus près dudit axe d'ouverture (54) que la première surface abrasive (26).

7. Dispositif selon l'une quelconque des revendications 1 à 6, dans lequel la pointe de tête de traitement (24) et la cavité (18) sont agencées pour former un composant intégré unique (46).

8. Dispositif selon l'une quelconque des revendications 1 à 7, dans lequel la seconde surface abrasive (28) est divisée en de multiples sections adjacentes (62) ayant chacune une caractéristique abrasive différente.

9. Dispositif selon l'une quelconque des revendications 1 à 8, dans lequel la seconde surface abrasive (28) comprend des objets tranchants (72) faisant saillie depuis ladite seconde surface abrasive (28).

10. Dispositif selon la revendication 9, dans lequel lesdits objets tranchants (72) font saillie depuis ladite surface et ont une longueur comprise entre 5 et 25 µm.

11. Dispositif selon l'une quelconque des revendications 1 à 10, comprenant en outre une source de vide (30) qui est connectée à l'ouverture (22).

12. Dispositif selon la revendication 11, comprenant en outre un dispositif de commande (38) qui est configuré pour commander la source de vide (30) afin d'appliquer une sous-pression alternative dans la cavité (18).

13. Dispositif selon l'une quelconque des revendications 1 à 12, dans lequel la paroi intérieure (22) comprend une section flexible (78).

14. Dispositif selon la revendication 13, dans lequel la seconde surface abrasive (28) est agencée sur ladite section flexible (78).
